(19) Europäisches Patentamt
European Patent Office
Office européen des brevets

(11) Publication number: 0 339 531 B1

# (12) EUROPEAN PATENT SPECIFICATION

(45) Date of publication of patent specification: **02.12.92**

(51) Int. Cl.[5]: **A61K 39/35**, A61K 39/36, A61K 47/00

(21) Application number: **89107314.0**

(22) Date of filing: **22.04.89**

Consolidated with 89904802.9/0402418
(European application No./publication No.) by
decision dated 06.06.91.

## (54) Physiological active liquid allergenic composition from biological raw materials.

(30) Priority: **27.04.88 DE 3814113**

(43) Date of publication of application:
**02.11.89 Bulletin 89/44**

(45) Publication of the grant of the patent:
**02.12.92 Bulletin 92/49**

(84) Designated Contracting States:
**AT BE CH DE ES FR GB IT LI LU NL SE**

(56) References cited:
**EP-A- 0 106 727**
**GB-A- 1 282 163**
**US-A- 4 226 853**

**JOURNAL OF ALLERGY AND CLINICAL IMMUNOLOGY, vol. 76, no. 4, October 1985, pages 543-550, St. Louis, US; E.M. VAN HOEYVELD et al.: "Stabilizing effect of epsilon-aminocaproic acid on allergenic extracts"**

**ANNALS OF ALLERGY, vol. 53, no. 5, November 1984, pages 426-431, Minneapolis, US; R. AYUSO et al.: "Stability of Lolium perenne extract"**

(73) Proprietor: **Tetra Consultants Inc.**
**P.O.Box 66 Wykagyl Station**
**New Rochelle, N.Y. 10804(US)**

(72) Inventor: **Stevens, Erich, c/o Lab. of Clinical Immunology**
**University Hospital St. Rafael, Kapucijnenvoer 33**
**B-3000 Leuven(BE)**
Inventor: **LeJoly, G. M., c/o Lab. of Clinical Immunology**
**University Hospital St. Rafael, Kapucijnenvoer 33**
**B-3000 Leuven(BE)**

(74) Representative: **Sternagel, Hans-Günther, Dr. et al**
**Patentanwälte Dr. Michael Hann Dr. H.-G. Sternagel Sander Aue 30**
**W-5060 Bergisch Gladbach 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 339 531 B1

## Description

The invention relates to physiological active, liquid allergenic compositions from biological raw materials and a process for the preparation of such compositions.

It has been recognized by the medical world for a long time that the physiological active components in liquid active compositions, in particular in active compositions of biological origin, tend to chemically degrade. The poor storing stability of such compositions makes it difficult if not impossible to be able to store them. The uncertain activeness of aged liquid preparations complicates their practical handling. In order to prevent risks the effectiveness always has to be tested before use. The diagnostic and therapeutical treatment is thus greatly hampered.

Physiologically active substances of biological origin, which up to now were prevented from being employed medically as liquid composition due to their chemical instability, include. antibiotics, hormones, steroids, toxins, sera, vaccines, allergenic substances, etc.

The poor storing stability, particularly as far as allergenic extracts from biologically allergenic sources, such as pollens are concerned, is disadvantageously noticeable in practical application due to the danger of wrong dosage. Liquid pharmaceutical active compositions from biologically allergenic sources play an important part as preparations for parenteral administration in the diagnosis and hypo- and desensibilization therapy of allergies. Due to the ever increasing symptoms in the field "allergy" intensive research into satisfactorily solving the problem of instability in allergenic compositions has been carried out, in particular for such, which are optionally parenterally applied, after having been specially treated.

Thus for example, in order to be able to provide sufficiently storable, marketable standard compositions of the known activity, it was suggested to coat the inner walls of the ampoules for receiving such liquid compositions with silicon. There was also mention of allergenic extracts made in phosphate or bicarbonate buffered salines, and the use of glycerin, polyoxyethylene(20)-sorbitanmonooleate, dextrose, serum albumin, etc. as stabilizing additive was suggested. An effective method of improving the storing stability of liquid compositions of physiologically active substances was described in US-A-4,600,583 and US-A-4,505,557 corresponding to EP-A-106727. Therein the stabilizing effect is obtained by adding aliphatic or alicyclic amino substituted carboxylic acids, resp. their pharmaceutically acceptable derivates or salts, whereby the carboxylic acid unit of the stabilizing compound exhibits a terminal, optionally an alkyl substituted amino group. By adding compounds such as tranexamic acid, lysine or ε-aminocaproic acid in small amounts, depending on the storage temperature (37 °C; 4 °C), it was possible to increase the storage stability by two to three times compared to allergenic extract compositions without the addition of such stabilisors. In examples I and II of US-A-4,605,557 a 0.1 M epsilon aminocaproic acid containing extraction solution having a pH-value of 7 was used for extracting the allergen of Lolium perenne pollen, to which a small amount of phenol was added as preservative.

E.A.M. Stevens et al. disclosed in J. Allergy Clinical Immunology Vol.76, No. 4, October 1985 a liquid allergenic composition extraced with,and stored in a solution comprising an additive of the formula

$$\begin{matrix} Y \searrow \\ \qquad N-(CH_2)-M(A)_nCOOX \\ Y \nearrow \end{matrix}$$

in which X and Y are independently hydrogen or $C_1$-$C_5$ alkyl group, M is an alkylene or cycloalkylene group, A is a substituted or unsubstituted methylene group and n is equal to 0 or 1 at a pH value of 7.

Nevertheless, at a storage temperature of 4 °C the activity (RAST score) decreased greatly within three months, namely, to approximately one fifth of the original score; at 37 °C such a decrease in activity was already reached after seven days.

Despite the improvements in the relative storage stability according to the teaching of the cited US-patents, the problem remained unsolved as to the retention of the absolute activity.

For medicinal purposes it is on the one hand necessary to provide active compositions, in particular allergenic compositions, whose activity remains unaltered over a greatest possible length of time, even at higher temperatures, and on the other hand there is also the necessity of such active compositions being available for a method of production, which, depending on the biological raw material, during extraction transfers the largest possible quantity of active substances at the highest possible concentration into the liquid phase. Of course, the resulting stable active composition should contain no substances, such as glycerin in high concentrations, which would hinder parenteral administration.

Thus, the object of the present invention is to provide even better storable liquid active compositions, in

particular allergenic compositions, depending on the biological raw material, which - optionally after further treatment - are suitable for parenteral administration, and to depict a method, which enables such active compositions to be extracted, whereby a wide spectrum of active components in high concentrations are retained.

It was completely unexpectedly found that a liquid allergenic composition obtained from extraction of biological raw materials in the presence of acid buffer system and having a pH value of between 5.5 and 6.5 and containing as an additive a compound of the formula I

$$\begin{matrix} Y \\ Y \end{matrix} \!\!> N-(CH_2)_n-MN-(A)_n-COOX \qquad (I)$$

whereby

- Y is H or $C_1$ to $C_5$-alkyl,
- n is equal to 0 or 1,
- M is an alkylene or cycloalkylene group, and
- A is a substituted or unsubstituted methylene group, and
- X is H or a $C_1$ to $C_5$ alkyl group or

pharmaceutically compatible salts.

The compositions are also storable at the extraction pH-values.

In the process according to the invention for preparing such an allergenic composition from biological raw materials, the biological raw material is extracted with an aqueous extraction solution, which contains as additive a compound of formula I or one of their pharmaceutically compatible salts. The characteristic of the process according to the invention consists of the extraction solution having a pH-value of between 5.5 and 6.5 controlled by a buffer system. Preferred pH-values are 6.0 and 6.5.

The particularly preferred additive from formula I is epsilon amino caproic acid.

The compound of the formula I is preferably employed at concentrations of between 0.001 M to 0.5 M. Particularly preferred is a solution of 0.1 M.

A very suitable buffer system is acetic acid/Na-acetate or phosphoric acid/alkali phosphate.

The pharmaceutical compositions according to the invention demonstrate an unexpectedly synergic participation of the additives according to formula I known from the prior art at common concentrations with the particular pH-value of the composition, which is maintained during extraction and the subsequent storage. It further shows that with the extraction pH-value according to the invention particularly important allergens can be extracted and stabilized, which would not have been possible with a neutral pH-value, although this range of pH-values is more toxic for allergenic proteins than the known pH-value of 7.

The extraction solutions can, apart from water, also comprise suitable solvents such as alcohols, e.g., ethanol.

Particularly suitable biological raw materials are plant pollens, particularly grass pollens.

Table I illustrates this effect for different allergenic extracts of plant pollens as raw material.

TABLE I

| biological sources | aqueous extraction solution with an extraction pH-value at 0.1M ε-amino caproic acid and stabilization of the pH-value with NaOH/acetic acid (acetic acid/Na-acetate) | |
|---|---|---|
| pollen | | |
| | storage pH | extraction pH |
| Lolium perenne | 6.5 | 6.5 |
| Phleum pratense | 5.5 | 5.5 |
| Dactylus glomerata | 5.5 | 5.5 |
| Anthoxantum odoratum | 6.5 | 6.5 |
| Betula verrucosa | 5.5 | 6.5 |
| Olea europaea | 6.5 | 6.5 |
| Parietaria judaica | 5.5 | 5.5 |
| Artemisia vulgaris | 5.5 | 5.5 |

Each extraction solution was produced with pyrogen-free destilled water. Each extraction was carried out under normal laboratory conditions and at room temperature or at 4°C. 100 mg or more pollen were always extracted over 1 to 21 h and the separation carried out by means of a centrifuge.
The fact that the stabilized active compositions according to the invention are not just a simple effect of the pH-value or the commonly known stabilizing effect of the substances according to formula I, is illustrated by the following experiments, which prove the synergetic effect.

Materials and methods:

raw materials: the commercially available, 99% pure Lolium perenne pollen from HAL Laboratories, The Netherlands was used.

Extraction method:

All the extracts were made at room temperature (+/- 23°C). Five hundred mg of Lolium perenne pollen were extracted with 4 ml of extracting fluid during 1 hour under constant vibration (300 vpm). The mixture was centrifuged at 1200 g for 20 minutes at 4°C. The supernatant was then recovered and centrifuged for 10 minutes at the same speed, and passed over a Millex(R)-GV 0.2 µm filter to prevent bacterial growth during storage. protein content was determined initially for several extracts by the Lowry method and was found to be constant between 8 and 9 mg/ml.

Buffer solutions:

Buffer solutions were made according to standard laboratory methods. A pH-control for the pH-stabilization during the extraction and for monitoring the pH-value for storage of 0.1 NaOH and 1 N-acetic acid (acetic acid/Na-acetate) was added by an automatic system (pH-Stat) consisting of an E 632 Digital pH meter, a 614 Impulsomat and a 665 Dosimat(R) from METROHM, Switzerland.
This system keeps the pH constant with a maximal deviation of 0.01 pH unit.

It was found that the adjusted pH value varied lower than 0. 5 pH units under the storage conditions used in this study (21 hours at 37°C).

Determination of content and activity:

Isoelectric focusing (IEF)

This method was performed on a 1% LITEX(R) agarose gel of 0.5 mm on Gelbond(R) in an ampholyte mixture, pH range 3.5 to 10, and appropriate electrode solutions. The sample was applied in an paratex (R) paper (20 µl of sample on a paper of 10 to 5 mm) placed at 2 cm from the cathode.

The IEF run was carried out at 7W for 900 volthours at 4°C. The paratex (R) paper was removed after 450 volthours.

Afterwards, the gel was fixed for 30 minutes in a solution containing 10% trichloroacetic acid and 5% sulfosalicylic acid. After two rinses in destaining solution (35% methanol, 10% acetic acid in water) the gel was dried and stained for 30 minutes with 0.2% coomassie brilliant blue R 250 solution of a composition similar to the washing fluid. The dry gels were scanned on a Helena Quick Scan Densitometer.

Isoelectric focusing and immunoblotting:

A print of the agarose gel was made on nitrocellulose according to the method described by Peltre (G. Peltre, B. David: Application of the nitrocellulose immunoprint technique to the allergen standardization. Regulatory control and standardization of allergenic extracts. Articles from the Paul Ehrlich Institute. Page 121, published by Gustav Fischer, Stuttgart, N.Y. 1984) The nitrocellulose sheet (Schleicher and Schull, BA 85,0.45 µm) was wetted in distilled water and applied on the agarose gel after covering it with water. Then the sandwich is covered with a pad of absorbing paper, a glass plate and 1 kg. weight for 5 minutes. Subsequently, the absorbing paper was replaced and the gel pressed under a 5 kg weight for 5 minutes. After drying, the nitrocellulose print was saturated with 3% boving serum albumin (BSA) in PBS for 1 hour at 45°C. The nitrocellulose was then incubated overnight at room temperature with the pollen sensitive patients' serum pool diluted 1/3 in PBS + 0.1% Tween(R)20 under constant agitation.

After 3 rinses in the dilution medium, the nitrocellulose was incubated for 2 hours with radio-labelled anti-IgE (Pharmacia) containing 20,000 - 40,000 cpm. After washing, protein bands reacting with IgE were visualized autoradiographically (Dupont Cronex(R)4-film, by incubation at -70°C for 4 days.

RAST-inhibition tests:

A pool of 50 sera containing high titers of IgE antibody against Lolium perenne was used as the source of antigen specific IgE. The sera were selected from samples received by the laboratory for routine RAST determination. They all gave a RAST score of + + + (five to 11 times the negative control) or + + + + (more than 11 times the negative control). Lolium perenne-coupled Pharmacia discs (Pharmacia Belga, Brussels. Belgium) were used for routine testing. For a negative control of about 200 cpm, the serum pool scored 40 to 50 times higher.

RAST-inhibition tests were carried out as follows:

50 µl of the allergenic extract was incubated with 50 µl of the serum pool. After 3 hours Lolium perenne-coupled discs were added to the tubes. For this purpose Lolium perenne extract (10 mg dry weight) was coupled to 1 gm of CNB-activated Whatman cellulose paper discs according to the method of Ceska et al (J. Allergy Clin. Immunol. 49, 1, 1972).

The insufficient stabilization during extraction and after the subsequent storage of 1 day at 37°C at pH-values of between 5.5 and 8.5 can be derived from Fig. 1. In accordance with the teaching of US-A-4, 605, 557 a pH-value of 7 - 7.6 appears optimal, since an activity potency of approximately 40% is maintained, whereas at pH 5.5 only 10% of the original potency is maintained. At pit 6.5 32% of the original potency are maintained.

The life-time quotient in Fig. 2 of the active substance (remaining active potency) when epsilon amino caproic acid (0.1M) is employed and the stabilization by controlling the pH-value depending on the pH-value returned (after storing for 1 day at 37°C). Whereas at the optimal pH-value of 7.0 to 7.5 the quotient lies between 2.3 and 2, the value in the acid range increases noticeably to between 5.5 and 6.5, at pH 6.5 it already reaches 3.5 at pH 6.0 5.8 and at pH 5.5 9.5. This result was completely unexpected, since in the slightly acid range, based on Fig. 1 actually a lower stability was to have been expected, in particular since in this pH range greater denaturization symptoms of the protein were to have been expected.

The storing stability of extracts of Lolium perenne pollen at extraction and storing pH 5.5 with and without epsilon amino caproic acid (0.1M):

| | % of potency | |
|---|---|---|
| | without amino caproic acid | with amino caproic acid |
| 1 day at 37°C | 15% | 46% |
| 6 months at 4°C | 20% | 42% |

In further RASTinhibition tests each of the extraction pH-values was maintained during storage. The measuring of the activity was carried out as already described.

A Lolium perenne extract in distilled water was adjusted to a certain storage pH value by means of a pH Stat to which (NaOH/acetic acid) and $\epsilon$-aminocaproic acid in concentrations of 0.1 M were added.
The average was formed by three such tests.

TABLE II.

| Sample | pH | stored for 1 day at | |
|---|---|---|---|
| | | 4°C average | 37°C average |
| a | 5 | 1.01 | 0.98 |
| b | 7.8 | 1.26 | 0.76 |
| c | 6.5 | 1.56 | 1.07 |
| d | 6.0 | 1.45 | 1.04 |

The superior activity of samples c and d at both temperatures can be derived from table II.
There was no alteration in the pH value of the adjusted extracts during storage.

Extraction conditions:

IEF analysis show that a buffer solution on a PBS-basis has a detrimental effect on the quality of the extract. / The capacity of the buffer, even at high concentrations of all common buffers (up to 0.5 M) is insufficient for keeping the pH-value constant.

The effects of the pH-value on the active content of fresh extracts in pH stabilized extracts with NaOH/acetic acid in destilled water are demonstrated by means of the following IEF-patterns (Fig. 3).

The $\epsilon$-aminocaproic acid content in extraction mediums is 0.1 M.
The superior quality maintenance with regard to the active substances in extraction pH values of 5.6 and 6.5 is illustrated in Fig. 3.

Similar results have been acchieved with the other biological sources of table I.

**Claims**

**1.** A liquid allergenic composition obtained from extraction of biological raw materials in the presence of acid buffer system and having a pH value of between 5.5 and 6.5 and containing as an additive a compound of the formula I

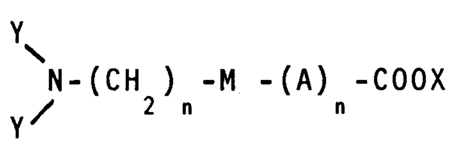

(I)

whereby

Y is H or $C_1$ to $C_5$-alkyl,
n is equal to 0 or 1,
M is an alkylene or cycloalkylene group, and

A is a substituted or unsubstituted methylene group, and
X is H or a $C_1$ to $C_5$ alkyl group or
pharmaceutically compatible salts.

**2.** Composition according to claim 1,
**characterized in that**
it comprises as additive of formula I ε-aminocaproic acid.

**3.** Composition according to claims 1 and 2,
**characterized in that**
the buffer system is acetic acid/Na-acetate.

**4.** Composition according to claims 1 to 3
**characterized in that**
its pH value is adjusted to 6.0.

**5.** Composition according to claims 1 to 4
**characterized in that**
it comprises a compound of the formula I at a concentration between 0.001 and 0.5 M, preferably 0.1 M.

**6.** Process for the preparation of an allergenic composition by extracting biological raw materials in an aqueous extraction solution, comprising a compound of the formula

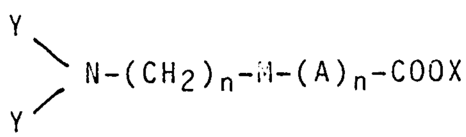

(I)

whereby
Y is H or $C_1$ to $C_5$-alkyl,
n is equal to 0 or 1,
M is an alkylene or cycloalkylene radical, and
A is a substituted or unsubstituted methylene group,
X is H or a $C_1$ to $C_5$-alkyl group
or its pharmaceutically compatible salt,
**characterized in that**
during the extraction the pH value of the extraction solution is maintained at a value of between 5.5 and 6.5 with a buffer system.

**7.** Process according to claim 6
**characterized in that**
acetic acid/Na-acetate is employed as buffer system.

**8.** Process according to claims 6 and 7,
**characterized in that**
the pH-value is maintained at 6.0 during the extraction.

**9.** Process according to claims 6 to 8,
**characterized in that**
the extraction solution comprises ε-aminocaproic acid as additive of the formula (I).

**10.** Process according to claims 1 to 9,
**characterized in that**
the additive of the formula (I) is comprised in the extraction medium at a concentration of between 0.001 M and 0.5 M.

## Patentansprüche

1. FLüssige Allergenzusammensetzung, erhalten durch Extraktion biologischer Rohstoffe in Gegenwart eines sauren Puffersystems und mit einem pH-Wert zwischen 5,5 und 6,5, und die als Zusatz eine Verbindung der Formel I

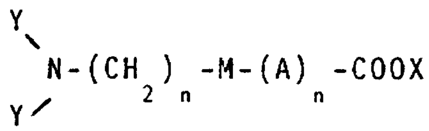

(I),

in der

Y Wasserstoff oder $C_1$-$C_5$-Alkyl ist,
n gleich 0 oder 1 ist,
M eine Alkylen- oder Cycloalkylengruppe ist und
A eine substituierte oder unsubstituierte Methylengruppe ist und
X Wasserstoff oder eine $C_1$-$C_5$-Alkylgruppe ist, oder deren pharmazeutisch verträgliche Salze enthält.

2. Zusammensetzung nach Anspruch 1,
**dadurch gekennzeichnet,**
daß sie als Zusatz der Formel I ε-Aminocapronsäure enthält.

3. Zusammensetzung nach Ansprüchen 1 oder 2,
**dadurch gekennzeichnet,**
daß das Puffersystem Essigsäure/Natriumacetat ist.

4. Zusammensetzung nach Ansprüchen 1 bis 3,
**dadurch gekennzeichnet,**
daß ihr pH-Wert auf 6 eingestellt ist.

5. Zusammensetzung nach Ansprüchen 1 bis 4,
**dadurch gekennzeichnet,**
daß sie eine Verbindung der Formel I in einer Konzentration zwischen 0,001 und 0,5 M, vorzugsweise 0,1 M enthält.

6. Verfahren zum Herstellen einer Allergenzusammensetzung durch Extrahieren biologischen Rohmaterials mit einer wäßrigen Extraktionslösung, enthaltend eine Verbindung der Formel

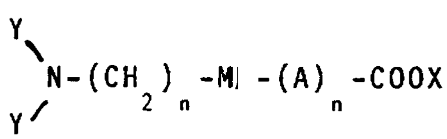

(I),

in der

Y Wasserstoff oder $C_1$-$C_5$-Alkyl ist,
n gleich 0 oder 1 ist,
M eine Alkylen- oder Cycloalkylengruppe ist und
A eine substituierte oder unsubstituierte Methylengruppe ist,
X Wasserstoff oder eine $C_1$-$C_5$-Alkylgruppe ist

oder deren pharmazeutisch verträgliche Salze,
**dadurch gekennzeichnet,**
daß während der Extraktion der pH-Wert der Extraktionslösung mit einem Puffersystem auf einem Wert zwischen 5,5 und 6,5 gehalten wird.

7. Verfahren nach Anspruch 6,

**dadurch gekennzeichnet,**
daß Essigsäure/Natriumacetat als Puffersystem verwendet wird.

**8.** Verfahren nach Ansprüchen 6 und 7,
**dadurch gekennzeichnet,**
daß der pH-Wert während der Extraktion bei 6,0 gehalten wird.

**9.** Verfahren nach Ansprüchen 6 bis 8,
**dadurch gekennzeichnet,**
daß die Extraktionslösung $\epsilon$-Aminocapronsäure als Zusatz der Formel (I) enthält.

**10.** Verfahren nach Ansprüchen 1 bis 9,
**dadurch gekennzeichnet,**
daß der Zusatz der Formel (I) in dem Extraktionsmedium in einer Konzentration zwischen 0,001 M und 0,5 M enthalten ist.

**Revendications**

**1.** Composition allergénique liquide obtenue par extraction de matériaux bruts biologiques en présence d'un système tampon acide et ayant une valeur de pH comprise entre 5,5 et 6,5 et contenant comme additif un composé de formule (I) :

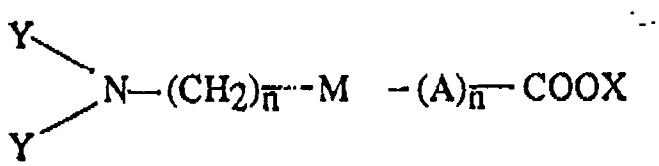

(I)

dans laquelle
Y est H ou un alkyle $C_1$-$C_5$,
n est égal à 0 ou 1,
M est un groupe alkylène ou cycloalkylène et,
A est un groupe méthylène, substitué ou non, et
X est H ou un groupe alkyle en $C_1$-$C_5$ ou des sels pharmaceutiquement compatibles

**2.** Composition selon la revendication 1, caractérisée en ce qu'elle comprend comme additif de formule (I) l'acide $\epsilon$-aminocaproïque.

**3.** Composition selon les revendications 1 et 2, caractérisée en ce que le système tampon est acide acétique/acétate de sodium.

**4.** Composition selon les revendications 1 à 3, caractérisée en ce que sa valeur de pH est ajustée à 6,0.

**5.** Composition selon les revendications 1 à 4, caractérisée par le fait qu'elle comprend un composé de formule (I) à une concentration comprise entre 0,001 et 0,5 M, de préférence 0,1 M.

**6.** Procédé de préparation d'une composition allergénique par extraction de matériaux bruts biologiques dans une solution d'extraction, comprenant un composé de formule (I) :

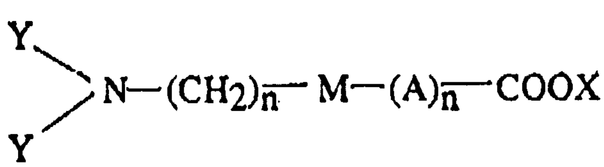

(I)

dans laquelle

Y est H ou un alkyle en $C_1$-$C_5$,
n est égal à 0 ou 1,
M est un radical alkylène ou cycloalkylène, et
A est un groupe méthylène, substitué ou non,
X est H ou un groupe alkyle en $C_1$-$C_5$
ou un sel pharmaceutiquement compatible,
caractérisé par le fait que pendant l'extraction, la valeur du pH de la solution d'extraction est maintenue à une valeur comprise entre 5,5 et 6,5 au moyen d'un système tampon.

**7.** Procédé selon la revendication 6, caractérisé en ce qu'on utilise comme système tampon acide acétique/acétate de sodium.

**8.** Procédé selon les revendications 6 et 7, caractérisé en ce que la valeur de pH est maintenue à 6,0 pendant l'extraction.

**9.** Procédé selon les revendications 6 à 8, caractérisé en ce que la solution d'extraction comprend comme additif de formule (I), l'acide $\epsilon$-aminocaproïque.

**10.** Procédé selon les revendications 1 à 9, caractérisé en ce que l'additif de formule (I) se trouve dans le milieu d'extraction à une concentration comprise entre 0,001 M et 0,5 M.

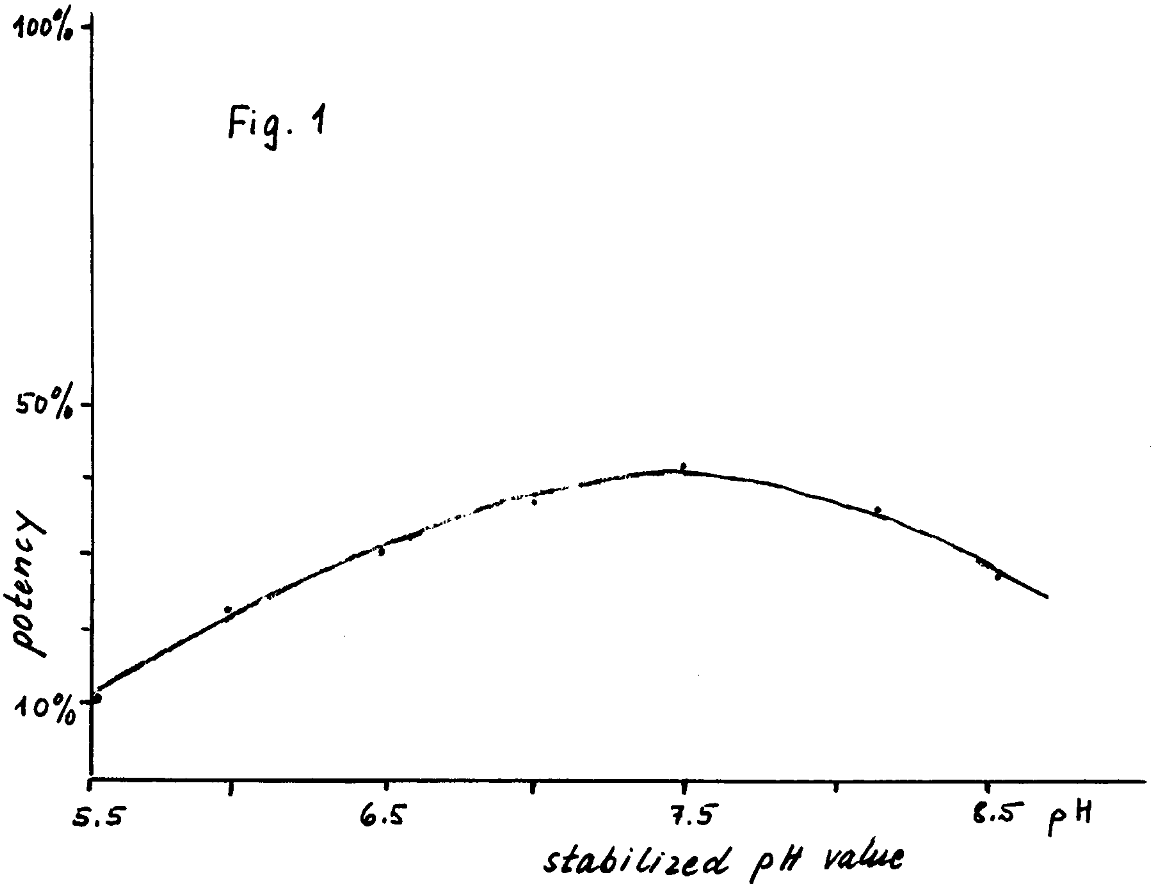
100%
Fig. 1
50%
potency
10%
5.5
6.5
7.5
8.5
pH
stabilized pH value

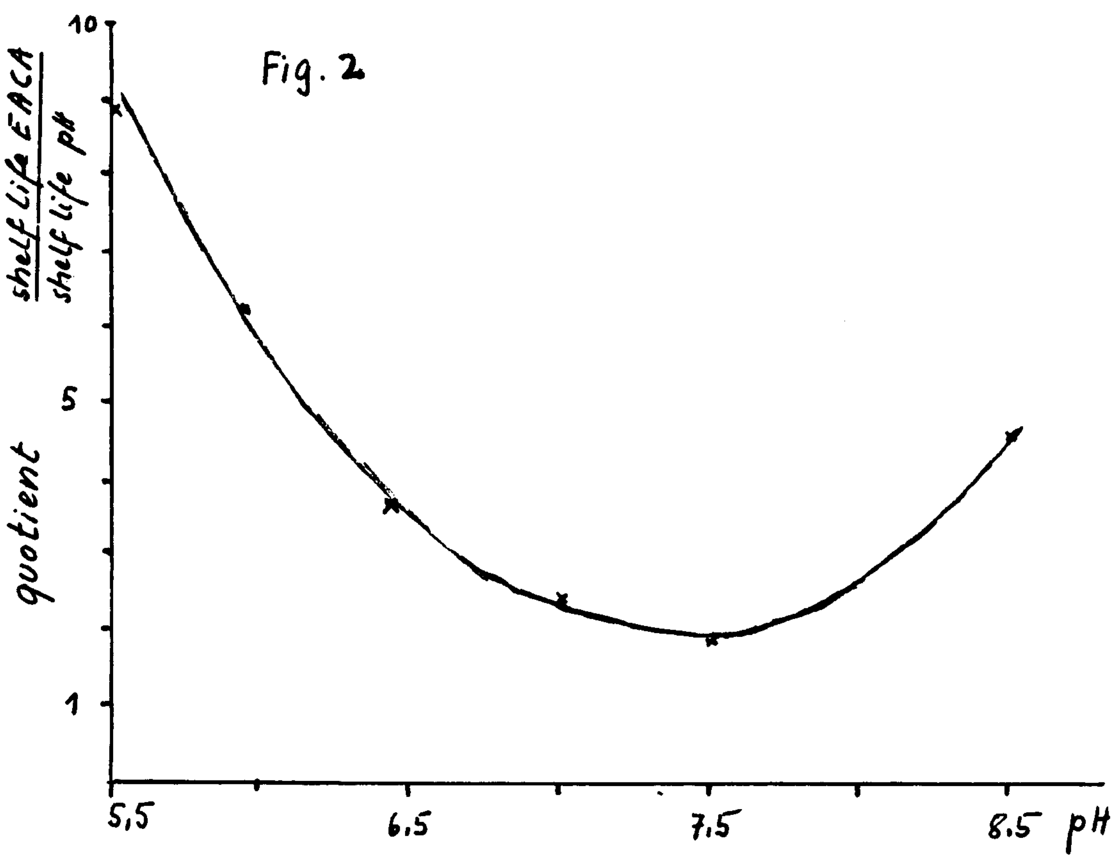
10
Fig. 2
shelf life EACA / shelf life pH
5
quotient
1
5,5
6,5
7,5
8,5
pH

Fig. 3:

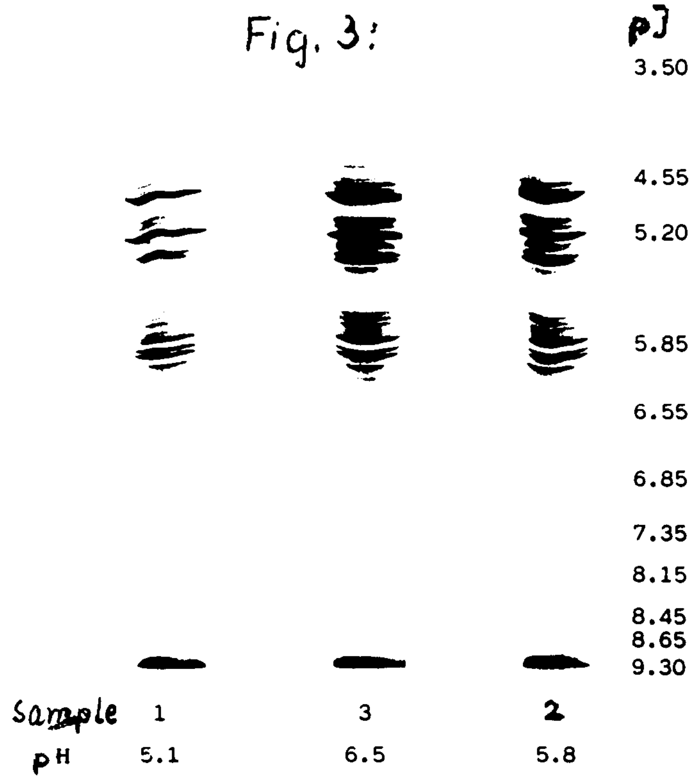


Fresh extracts